# EUROPEAN PATENT SPECIFICATION

(11) **EP 3 833 695 B1**
(45) Date of publication and mention of the grant of the patent: **07.01.2026**
(21) Application number: 19746507.3
(22) Date of filing: 06.08.2019
(51) Int. Cl.: C08B 37/00, A61L 27/52, A61K 31/167, C08B 37/08, C08L 5/08

(54) **METHOD FOR DYNAMIC FILTRATION OF A CROSS-LINKED HYDROGEL**
VERFAHREN ZUR DYNAMISCHEN FILTRIERUNG EINES VERNETZTEN HYDROGELS
PROCÉDÉ DE FILTRATION DYNAMIQUE D'UN HYDROGEL RÉTICULÉ

(30) Priority: 07.08.2018 WO PCT/EP2018/071328
(43) Date of publication of application: 16.06.2021
(73) Proprietor: Merz Pharma GmbH & Co. KGaA, 60318 Frankfurt am Main (DE)
(72) Inventor: PFEIL, Michael, 60318 Frankfurt am Main (DE); KESSLER, Wolfgang, 60318 Frankfurt am Main (DE); CONRAD, Manuel, 60318 Frankfurt am Main (DE); NIEMCZAK, Björn, 60318 Frankfurt am Main (DE); VUKOVIC, Patrik, 60318 Frankfurt am Main (DE)
(74) Representative: Jacobi, Markus Alexander
(86) International application number: PCT/EP2019/071096
(87) International publication number: WO 2020/030629

(56) References cited:
- EP-A2- 2 614 828
- WO-A1-00/47312
- WO-A1-2009/080220
- RU-C1- 2 582 702
- US-A1- 2010 028 437
- S WROFISKI ET AL: "Dynamic filtration in biotechnology", BIOPROCESS ENGINEERING, vol. 4, no. 3, 1 April 1989 (1989-04-01), pages 99 - 104, XP055582266

## Description

### FIELD OF THE INVENTION

The present invention relates to a method for dynamic filtration of a cross-linked biopolymer-based hydrogel to remove unwanted molecules from the gel. In particular, the invention relates to dynamic filtration of a hyaluronic acid hydrogel using a dynamic filtration construction with rotating and semipermeable filter discs.

### BACKGROUND OF THE INVENTION

Hyaluronic acid is a polymer natural to the body, which since some time is employed in medicine in different fields such as in orthopedics and in ophthalmology. Nowadays, hyaluronic acid is increasingly used in aesthetic medicine and in plastic surgery. The broad application of hyaluronic acid is particularly due to its very high binding capability of water. In aqueous medium, even at low concentration of hyaluronic acid, viscoelastic gels are formed, which are biologically degradable, and which have advantageous properties.

Pure hyaluronic acid is relatively fastly degraded in the human body. For this reason, hyaluronic acid molecules are often chemically cross-linked with one another, whereby the degradation is severely decreased, and the desired effects are maintained for a period of about six to twelve months. Thus, cross-linked hyaluronic acid gels are, among others, frequently used in the aesthetic medicine for the treatment of wrinkles, contouring and volume increase of the upper and lower lip, the improvement of the contours of the cheeks and chin, and for corrections of the nose, etc.

The known hyaluronic acid gels can be roughly classified in two different types, namely mono-phased and bi-phased gels. The mono-phased hyaluronic acid gels consist of a single phase and are non-particulate. The preparation of such mono-phased gels is disclosed, for example, in WO 2008/068297, US 8,450,475, US 8,455,465, US 7,741,476, and US 8,052,990. Known commercial mono-phased hyaluronic acid gels are, for example, Juvéderm^{®}, Teosyal^{®}, Glytone Professional^{®}, and the mono-phased, double-cross-linked hyaluronic acid gels, which are known as Belotero^{®}, Esthélis^{®}, Fortélis^{®} Extra and Modélis^{®} Shape.

The bi-phased hyaluronic acid gels comprise a cross-linked hyaluronic acid material, which is dispersed in a liquid phase. Such gels are particulate and may be made as described in, for example, EP 0 466 300. Commercially available bi-phased gels are, for example, Hylaform^{®}, Restylane^{®}, and Perlane^{®}.

The making of cross-linked hyaluronic acid gels occurs by means of a multistep process and is described in more detail in WO 2005/085329.

Methods of making gels based on polysaccharides such as hyaluronic acid are further disclosed in WO 2014/064633, US 2013/210760, US 2013/203696, WO 2010/115081, WO 2012/062775, WO 2013/185934, WO 2009/077399 and WO 2008/034176.

Furthermore, RU 2 582 702 C1 discloses a method for preparing hyaluronate hydrogels, crosslinked with polyethylene glycol diglycidyl ether (PEDGE), wherein the hydrogels are subjected to tangential filtration.

The conventional methods for dialysis of hyaluronic acid gels frequently require a relatively high manual time and effort. This is insofar disadvantageous as quality and reproducibility are decreased and production time and costs are increased. Furthermore the risk of microbial contamination increases with process time of the dialysis step.

Frequently, 1,4-butanediol diglycidyl ether (BDDE) is used as a crosslinking agent for hyaluronic acid hydrogels. However, BDDE may be toxic and there is a demand to produce hydrogels having less unwanted molecules.

### OBJECT OF THE INVENTION

The present invention is based on the object to provide an improved method to remove unwanted molecules which are generated during production of a cross-linked biopolymer-based hydrogel.

### SUMMARY OF THE INVENTION

The invention is defined as set out in the independent claims and relates to a method for dynamic filtration of a cross-linked biopolymer-based hydrogel using a dynamic filtration device which is equipped with semipermeable filter disc(s) to remove unwanted molecules comprising the steps of i) concentrating the gel by applying a rotational speed within the range of 20 1/min to 500 1/min and a overpressure within the range of 0.5 to 6 bar to a predetermined concentration; or pumping the gel directly into the process chamber of the dynamic filtration device; ii) conducting a diafiltration to reduce unwanted molecules, selected from unreactive or unbound polymer cross-linker molecules and their degradation products, by applying a rotational speed within the range of 20 1/min to 500 1/min and a overpressure within the range of 0.5 to 6 bar, wherein diafiltration involves adding water or a new buffer to the retentate at the same rate as filtrate is being generated, wherein the biopolymer-based hydrogel is made of a polymer which is selected from the group consisting of hyaluronic acid, heparosan, alginate, pectin, gellan gum, chondroitin sulfate, keratan, keratan sulfate, heparin, heparin sulfate, cellulose, chitosan, carrageenan, xanthan, or salts or derivatives thereof or combinations thereof, and wherein the cross-linker bears at least two groups which are capable of reacting with one or more of the hydroxyl groups of the biopolymer-based hydrogel and to cross-link same.

### FIGURES

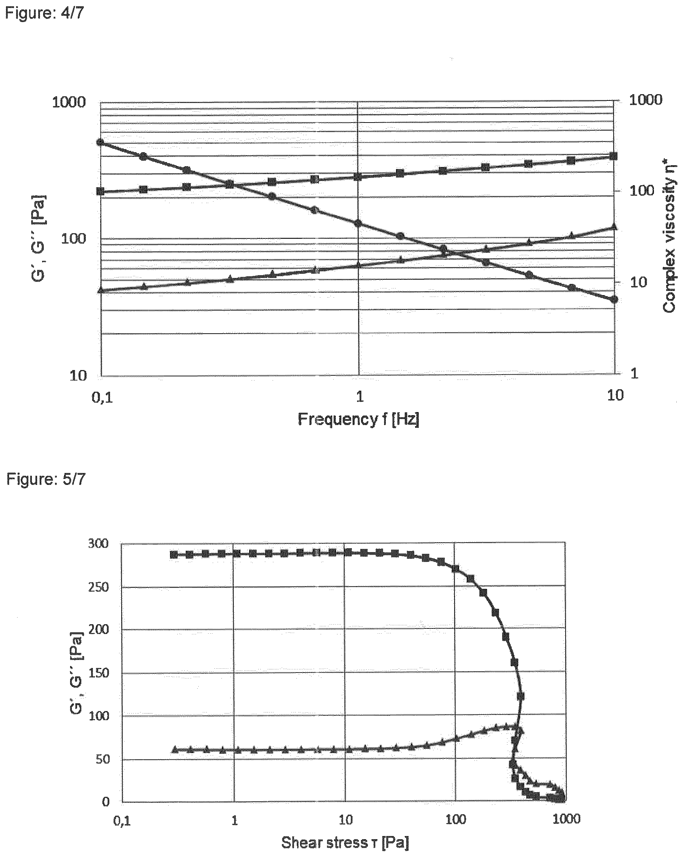

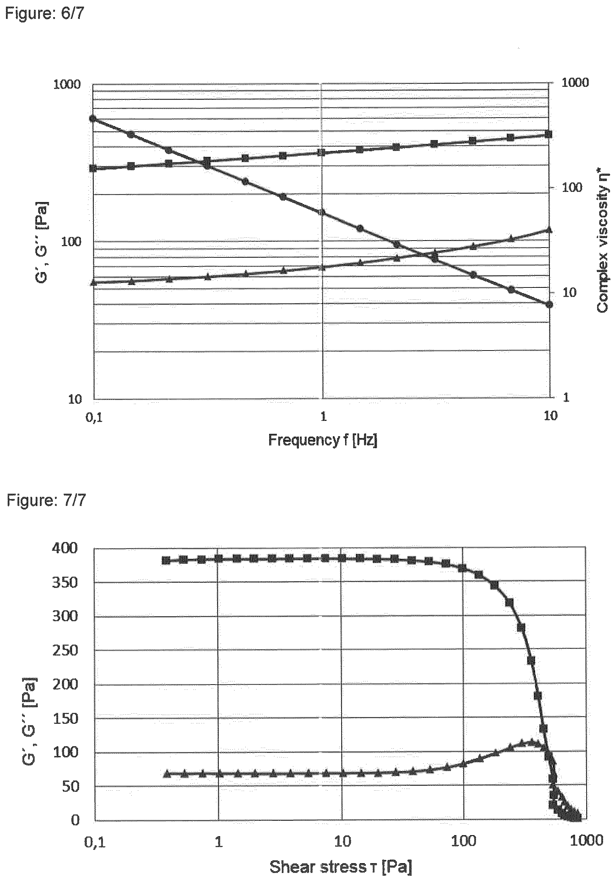

Figure 1: Frequency sweep comparison between a gel which is processed according to the invention ("DCF gel"; storage modulus (G') 200 Pa) and a gel which is processed according to a standard method ("standard gel"; storage modulus (G') 216 Pa).
   1 (dotted line) = DCF gel: G'
   2 (continuous line): standard gel: G'
   3 (dotted line) = DCF gel: G"
   4 (continuous line): standard gel: G"
   5 (dotted line) = DCF gel: complex viscosity
   6 (continuous line) = standard gel: complex viscosity
Figure 2: Amplitude sweep comparison between a gel which is processed according to the invention ("DCF gel"; storage modulus (G') 241 Pa) and a gel which is processed according to a standard method ("standard gel"; storage modulus (G') 249 Pa). Cross-over point = flow-point
   1 (dotted line) = DCF gel: G'
   2 (continuous line): standard gel: G'
   3 (dotted line) = DCF gel: G"
   4 (continuous line): standard gel: G"
Figure 3:
   1: DCF gel: G'
   2: DCF gel G"
   3: DCF gel: complex viscosity
Figure 4: Frequency sweep of the gel according to example 3 / trial 4
   continuous line with triangle ▲: loss modulus G"
   continuous line with square ▪: storage modulus G'
   continuous line with circle •: complex viscosity [η*]
Figure 5: Amplitude sweep of the gel according to example 3 / trial 4
   continuous line with triangle ▲: loss modulus G"
   continuous line with square ▪: storage modulus G'
   cross over point G"= G': shear stress 440 Pa; storage modulus 54 Pa
Figure 6: Frequency sweep of the gel according to example 3 / trial 5
   continuous line with triangle ▲: loss modulus G"
   continuous line with square ▪: storage modulus G'
   continuous line with circle •: complex viscosity [η*]
Figure 7: Amplitude sweep of the gel according to example 3 / trial 5
   continuous line with triangle ▲: loss modulus G"
   continuous line with square ▪: storage modulus G'
   cross over point G"= G': shear stress 350 Pa; storage modulus 98 Pa

### DETAILED DESCRIPTION OF THE INVENTION

The present invention may be understood more readily by reference to the following detailed description of the invention and the example included therein.

The term "dynamic filtration device" as used herein refers to a means using a hollow filter element in the form of a plate or disk which is arranged movable on a hollow shaft. By the relative movement of the element turbulence arise in the medium to be filtered, whereby the surface of the filter element is cleaned. Such a filter device is described for example in WO 00/47312. The rotating filter element allow for decoupling of overflow velocity from transmembrane pressure (TMP). The combination of centrifugal and shear forces in dynamic filtraton modules improves control of cover layer build-up. By using this technology the formation of a clogging layer is reduced. In addition, the relative movement ensures a mixing of the retentate in the filter machine. Such a dynamic filtration device can be obtained from different companies, for example from Andritz AG (Headquarters Graz Austria). The key element of the dynamic filtration device is the membrane disk. Its diameter, number of discs and alignment may vary depending on the intended scale.

According to the invention, the method of dynamic filtration can be applied on various cross-linked biopolymer-based hydrogels (also referred to as "hydrogel(s)" in the following). Methods for the production of cross-linked biopolymer-based hydrogels are well known in the art, for example, the making of cross-linked hyaluronic acid gels is described in WO 2005/085329 A1. Further methods of making gels based on polysaccharides are disclosed in WO 2014/064633, US 2013/210760, US 2013/203696, WO 2010/115081, WO 2012/062775, WO 2013/185934, WO 2009/077399 and WO 2008/034176.

In one aspect, the invention relates to a method for dynamic filtration of a cross-linked biopolymer-based hydrogel comprising the steps of
a) transferring a cross-linked biopolymer-based hydrogel in a dynamic filtration device which is equipped with semipermeable filter disc(s) and diafiltrating the gel comprising the steps of:
   i) concentrating the gel by applying a rotational speed within the range of 20 1/min to 500 1/min and a overpressure within the range of 0.5 to 6 bar to a predetermined concentration; or pumping the gel directly into the process chamber of the dynamic filtration device;
   ii) conducting a diafiltration to reduce unwanted molecules, selected from unreactive or unbound polymer cross-linker molecules and their degradation products, by applying a rotational speed within the range of 20 1/min to 500 1/min and a overpressure within the range of 0.5 to 6 bar;
b) optionally adding a mixture comprising a non-cross-linked polymer, in particular non cross-linked hyaluronic acid, and water to the gel,

wherein diafiltration involves adding water or a new buffer to the retentate at the same rate as filtrate is being generated,
wherein the biopolymer-based hydrogel is made of a polymer which is selected from the group consisting of hyaluronic acid, heparosan, alginate, pectin, gellan gum, chondroitin sulfate, keratan, keratan sulfate, heparin, heparin sulfate, cellulose, chitosan, carrageenan, xanthan, or salts or derivatives thereof or combinations thereof, and
wherein the cross-linker bears at least two groups which are capable of reacting with one or more of the hydroxyl groups of the biopolymer-based hydrogel and to cross-link same.

As used herein, the term "diafiltration" is used to mean a continuous diafiltration (also referred to as constant volume diafiltration) which involves washing out the original buffer salts or other low molecular weight species in the retentate (sample) by adding water or a new buffer to the retentate at the same rate as filtrate is being generated.

The term "unwanted molecules" as used herein refers to unreactive or unbound polymer cross-linker molecules (e.g. BDDE) and their degradation products.

Surprisingly, it has been found that the method according to the invention removes significantly unwanted molecules from the gel, while the structural stability of the gel was maintained in spite of the share stress arising from the rotating filter discs. In addition, it was surprisingly found that the required process time and required buffer for conducting the method according to the invention was significantly reduced in comparison to conventional dialyses. It is a further advantage of the method according to the invention that flux rates are increased (two to ten times) in comparison to conventional cross-flow filtrations and that the retentate can be higher concentrated. These advantages lead to an improved method to remove unwanted molecules from cross-linked and highly viscous hydrogels.

The biopolymer-based hydrogel is made of a biologically degradable polymer, selected from: hyaluronic acid, heparosan, alginate, pectin, gellan gum, chondroitin sulfate, keratan, keratan sulfate, heparin, heparin sulfate, cellulose, chitosan, carrageenan, xanthan, or salts or derivatives thereof or combinations thereof. The term "hyaluronic acid" is synonymously used with the term "hyaluronan".

Although in the following the present invention is described by means of a hyaluronic acid hydrogel, this does not mean that the disclosure is in any way limited to a hyaluronic acid hydrogel. Rather, any biopolymer-based hydrogel may be used in place of hyaluronic acid hydrogel, which are made of one of the above-mentioned polymers.

The biopolymer-based hydrogel is cross-linked by means of a chemical cross-linking agent and the hydrogel contains a surplus of the used chemical cross-linking agent. The surplus of the chemical cross-linking agent is removed from the hydrogel by the method according to the invention. In addition, other unwanted molecules may also be removed from the hydrogel by the method according to the invention.

The term "cross-linker" comprises all compounds bearing at least two groups, which are capable of reacting with one or more of the hydroxyl groups of the biopolymer-based hydrogel and to cross-link same. Suitable groups of the cross-linker for cross-linking may be selected from carboxyl, epoxide, halogen, vinyl, isocyanate, or protected isocyanate groups. Exemplary compounds, which may be used as cross-linker, are, for example, 1,4-butanediol diglycidyl ether (BDDE), polyvinylsilane (PVS), PEG-based crosslinking agents, divinyl sulfone (DVS). In particular embodiments, the method according to the invention removes unbound or surplus of 1,4-butanediol diglycidyl ether (BDDE) and / or other unwanted molecules from the hydrogel.

Preferably, the cross-linked biopolymer-based hydrogel (in particular the hydrogel made of hyaluronic acid) exhibits an initial concentration in the range of from 10 to 70 mg/g, preferably from 20 to 50 mg/g. In terms of the present invention the concentration of the hydrogel means the amount of the cross-linked biopolymer (given in mg) based on the total amount of the hydrogel (given in g). Typically, the initial concentration is the concentration of the hydrogel which is transferred in the dynamic filtration device in step a) and which is optionally concentrated or pumped directly into the process chamber in step i).

In another embodiment of the invention the hydrogel can be diluted (e.g. using a buffer solution applied in the diafiltration) before it is transferred into the dynamic filtration device, for example to facilitate the infilling, and preferably concentrated in optional step i) before diafiltration. Preferably, the hydrogel is concentrated in optional step i) starting from a concentration of the hydrogel in the range of from 3 to 30 mg/g, preferably from 5 to 20 mg/g, more preferably from 5 to 10 mg/g.

Preferably, the hydrogel is concentrated in optional step i) until the desired concentration is reached and at most to a maximum concentration of 70 mg/g. In particular the concentration of the hydrogel after the optional concentrating step i) is in the range of from 10 to 70 mg/g, preferably from 20 to 50 mg/g, more preferably from 20 to 35 mg/g.

Preferably, the diafiltration in step ii) is conducted at a concentration of the hydrogel in the range of from 10 to 70 mg/g, preferably from 20 to 50 mg/g, more preferably 20 to 35 mg/g, also preferably 23 to 70 mg/g. Typically, the concentration of the hydrogel (in particular the hydrogel made of hyaluronic acid) is maintained at nearly the same level during diafiltration step ii).

Preferably, the diafiltration in step ii) is conducted until the amount of unwanted molecules (such as BDDE) in the hydrogel is below the limit of quantification. For example the diafiltration in step ii) is conducted for a period of from 1 to 8 hours, preferably 2 to 6 hours.

Preferably, the diafiltration in step ii) is conducted using a buffer solution, preferably a phosphate buffer solution, e.g. a buffer solution based on sodium phosphate, such as Na₂HPO₄ and NaH₂PO_{4.} In particular the diafiltration in step ii) is conducted using a buffer solution having a pH value in the range of 6 to 8, preferably 6.5 to 7.5.

Preferably the hydrogel is concentrated in step i) by applying a rotational speed in the range of from 70 1/min to 500 1/min, preferably from 70 1/min to 300 1/min, more preferably from 100 1/min to 300 1/min. Preferably the hydrogel is concentrated in step i) by a overpressure in the range of from 1 to 3 bar.

Preferably the diafiltration in step ii) is performed by applying a rotational speed in the range of from 70 1/min to 500 1/min, preferably from 70 1/min to 300 1/min, more preferably from 100 1/min to 300 1/min. Preferably the diafiltration in step ii) is performed by applying a overpressure in the range of from 1 to 3 bar.

In particular embodiments, the dynamic filtration device which is used in the method according to the invention is equipped with semipermeable filter disc(s) exhibiting a pore size of 5 nm to 2µm, more particularly a pore size of 30 nm to 600 nm, more particularly a pore size of 80 nm to 300 nm, particularly a pore size of 5 nm to 60 nm.

In particular embodiments, the dynamic filtration device which is used in the method according to the invention is equipped with one or more semipermeable filter disc(s). In a preferred embodiment the dynamic filtration device is equipped with 1 to 10, preferably 4 to 6, semipermeable filter disc(s). The semipermeable filter discs may exhibit the same or different dimensions. It was surprisingly found that the number of semipermeable filter disc(s) can be increased up to 10 or preferably up to 6, without having negative impact on the viscosity and cross-linked structure of the hydrogel. Typically, shear forces increase with increasing number of discs and increasing rotational speed.

In particular embodiments, the dynamic filtration device is equipped with semipermeable filter disc(s) which are made of ceramic, metal or polymer material.

In particular embodiments, the biopolymer-based hydrogel is made of hyaluronic acid and the gel is concentrated by applying a rotational speed within the range of about 20 1/min to 150 1/min and a pressure within the range of about of about 0.5 to 2 bar to a final concentration of 10 to 30 mg/g and wherein the diafiltration is performed by applying a rotational speed within the range of 20 1/min to 150 1/min and a pressure within the range of 0.5 to 2 bar.

In a preferred embodiment, the biopolymer-based hydrogel is made of hyaluronic acid, the diafiltration in step ii) is performed by applying a rotational speed within the range of from 20 1/min to 500 1/min, preferably from 70 to 300 1/min and a pressure within the range of from 0.5 to 3 bar, preferably from 1 to 3 bar, and the diafiltration in step ii) is conducted at a concentration of the hydrogel in the range of from 10 to 70 mg/g, preferably from 20 to 50 mg/g, more preferably from 20 to 35 mg/g.

In a preferred embodiment, the biopolymer-based hydrogel is made of hyaluronic acid, the gel is pumped directly into the process chamber of the dynamic filtration device in step (i) (i.e. no optional concentrating step) and the diafiltration in step ii) is performed by applying a rotational speed within the range of 70 1/min to 500 1/min, preferably from 70 to 300 1/min, and a pressure within the range of 1 to 3 bar, wherein the diafiltration in step ii) is conducted at a concentration of the hydrogel in the range of from 10 to 70 mg/g, preferably 20 to 35 mg/g, and wherein the dynamic filtration device is equipped with 1 to 10, preferably 4 to 6, semipermeable filter disc(s).

During or after diafiltration of the hydrogel further substances may be added to the gel, for example: salts, buffer substances, vitamins (e.g. vitamin **E,** C, B6), antioxidant (e.g. ascorbic acid or derivatives thereof, zinc oxide), polyols (e.g. glycerol, mannitol), tri-calcium phosphate particles (e.g., alpha- and beta-tri-calcium phosphate and hydroxyapatite particles), agents (e.g. anesthetics, anti-inflammatory agents, stimulus-reducing agents, vasoconstrictive agents or vasodilatory agents, anticoagulants, humidity-providing substances, immuno-suppressives, antibiotics, etc.) as well as growth factors, peptides or proteins (e.g. neurotoxins). **In** particular, an anesthetic such as lidocaine may be added to the gel. Preferably, lidocaine, is contained in the gel in a concentration of from 0.05 to 5.0 wt.-%, 0.1 to 2.0 wt. %, 0.1 to 1.0 wt.-%, 0.1 to 0.5 wt.-%, 0.1 to 0.4 wt.-%, 0.2 to 0.4 wt.-% or from 0.2 to 0.3 wt.-%, based on the total weight of the composition.

Preferably the optional concentrating in step i) and/or the diafiltration in step ii) is performed at a temperature in the range of 15 °C to 80 °C, preferably 20 °C to 70 °C.

In a preferred embodiment the diafiltration in step ii) and optionally the concentration step i) are performed at a temperature in the range of from 60 °C to 70 °C, preferably at a temperature in the range of from 60 °C to 70 °C and for a time period of from 2 to 4 hours (e.g. 3.5 hours). In particular a temperature in the range of 60 °C to 70 °C is advantageous due to the decreased viscosity of the hydrogel.

In particular embodiments, in step a) the gel is additionally undergoing a thermal treatment to reduce unwanted molecules, wherein 60°C are applied for up to four hours.

The dynamic filtration device may comprise a sterilization means, preferably pure steam, for rinsing the device and/or the passage with steam, a sterilization fluid, in particular washing liquid, preferably filtered water, and/or sterilized/particle-free air. The sterilization means may comprise a supply unit comprising sterilization fluid, and a storage unit for sterilization fluid.

In particular embodiments the gel is sterilized in the dynamic filtration device at 121 °C to 135°C with a holding time of about 2 to 30 minutes.

In particular embodiments, the method according to the invention is conducted in less than 10 hours, in particular in less than 5 hours.

The method according to the invention may additionally comprise the following steps:
i) degassing the gel to remove air bubbles under vacuum (20 to 200 mbar),
ii) filling the gel into syringes,
iii) sterilize the gel in the syringe by heat in an autoclave.

In another aspect the present invention relates to a cross-linked biopolymer-based hydrogel obtainable by the method according to the invention, wherein the amount of unreactive or unbound cross-linker molecules and their degradation products in the hydrogel is below the limit of quantification, measured by gas chromatography, wherein the cross-linker is BDDE.

In another aspect the present invention relates to a use of the cross-linked biopolymer-based hydrogel obtained by the method according to the invention for aesthetic applications, in particular soft tissue augmentation.

### EXAMPLES

In the following, the method according to the invention is explained in more detail.

### Example 1: Method including step of diluting, concentrating and diafiltration of the gel

A hyaluronic acid hydrogel which was cross-linked with BDDE (cf. for example WO 2005/085329) and exhibits an initial concentration of 24 mg/g was diluted in a range between 1:4 to 1:8 using a buffer containing Na2HPO4*2H2O: 0,994 g/L; NaH2PO4*2H2O: 0,51 g/L; Mannitol: 42 g/L and water for injection. The diluted gel was transferred to a container, connected to a dynamic filtration device inlet via a pipe or tubing. The dynamic filtration device consisted of a Krauss Maffei Dynamic Crossflow Filter DCF 152/S (Andritz AG) having one filter disc (0,034m² filter area, 152mm diameter and a pore size of 7 nm), one container as gel reservoir and a second container as buffer reservoir. Both containers were connected to the inlet port of the DCF 152/S via tubing and ball valve to select the respective medium in the different process steps. The double jacket of the housing of the DCF 152/S was connected to a refrigerated circulator to maintain the temperature in the double jacket at a preset temperature of 20°C. The filtrate of the DCF 152/S was collected in a container, placed on a balance to determine the amount of filtrate. The following steps were applied:
i) concentrating the gel by applying pressure (1,5 bar) to the closed gel container, containing the diluted gel, open the way to the inlet port of the DCF 152/S and removing the filtrate via the filter discs and hollow shaft (see WO 00/47312). The filter disc was rotating with 150rpm. The gel was concentrated until the concentration of hyaluronic acid in the gel was 24 mg/g.
ii) conducting a diafiltration with a buffer solution containing Na₂HPO₄*2H₂O: 0,994 g/L; NaH₂PO₄*2H₂O: 0,51 g/L; Mannitol: 42 g/L and water for injection by applying pressure (1,5 bar) to the closed buffer container, containing the buffer, open the way to the inlet port of the DCF 152/S and removing the filtrate via the filter discs and hollow shaft (see WO 00/47312). The buffer solution may contain lidocaine (0,27 - 0,33 *w*/*w%).* As an alternative, lidocaine may be added after diafiltration. The filter disc was rotating with 150rpm. The gel was filtered until BDDE is present below the limit of quantification.

The amount of BDDE was determined according to the following method:
1. Sample preparation:
   a. Enzymatic degradation of HA
      HA was degraded by addition of hyaluronidase solution and subsequent incubation for 1 to 4 hours under slight shaking until viscosity was reduced.
   b. Extraction of BDDE
      BDDE was extracted from the degraded sample (described under a) by addition of ethylacetate (5 % v/v) and orbital shaking for 20 min at 400 rpm. The solution was centrifuged at 10 min at 5000 rpm to separate the organic and aqueous phase. The organic phase, containing the BDDE was transferred to a GC-vial.
2. Gaschromatographic determination
   a. 5 µL of the sample (described under 1 b) was injected in a GC (split injector, split ration 1:5, 250°C) and separated on a DB-1 column (Agilent DB-1, 30 m, 0.25 mm ID, 0.25 µm film) using a temperature ramp 100°C - 40°C/min - 280°C and final holding at 280°C for 30 sec. A FID was used as detector.
   b. Data evaluation was performed by an internal standard method according to European Pharmacopoeia Chapter 2.2.46.
The result of one experiment according to Example 1 is shown in the following table 1:

**Table 1:**

| Disc [nm] | Pressure [bar] | T [°C] | Duration [h:m] | BDDE Gel BDDE content at beginning [ppm] | BDDE DCF Final BDDE content [ppm] |
|---|---|---|---|---|---|
| 7 | 1.5 | 20 | 6:00 | > 150 | below limit of quantification (LOQ) |

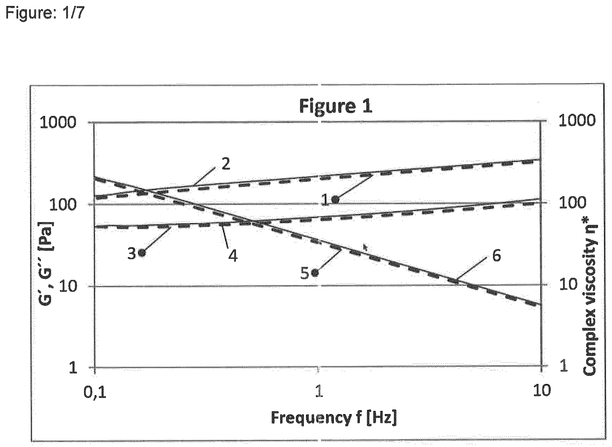

The structural stability of a gel was maintained during the method according to the invention as can be seen from Figure 1 which shows a comparison of two different gels. One gel was treated according to the invention ("DCF gel") and the other gel was treated with a standard method ("standard gel").

The continuous lines represent the characteristic progression of G', G" and η of a gel after dialysis which was produced according to a standard method, whereas the dotted lines represent the characteristic progression of a gel which was processed according to the invention.

It is apparent that there were no significant differences regarding the curve progressions shown in the frequency test which is commonly used for the characterization of gels. The curves related to the gel according to the invention are slightly below the curves related to the standard gel because the HA-concentration of the gel is slightly lower. With regard to the structural stability of the gel, the parallel increase of G' and G" (G' > G") clearly indicates that the gel has maintained its cross-linked structure during the method according to the invention.

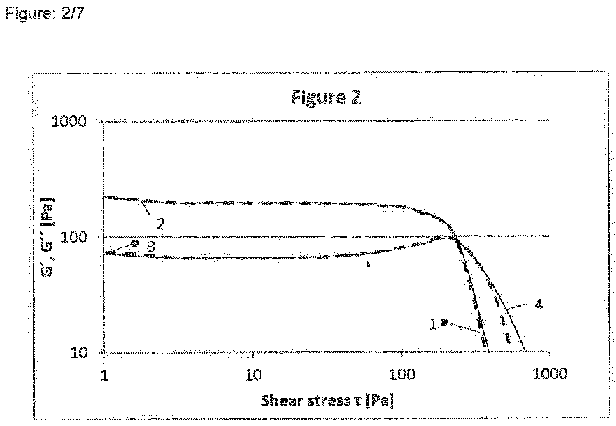

In addition, it is shown in Figure 2 that the flow-point of both gels ("DCF gel" and standard gel"; see above) is identical which further indicates that structural stability of the gel processed according to the invention was maintained.

### Example 2: Method of diafiltrating the gel without step of diluting the gel

A hyaluronic acid hydrogel which was cross-linked with BDDE (cf. for example WO 2005/085329) and exhibits a concentration of 32-35 mg/g was pumped directly into the process chamber of the dynamic filtration device. The gel contained 226.8 ppm unbound BDDE. The dynamic filtration device consisted of a Krauss Maffei Dynamic Crossflow Filter DCF 152/S (Andritz AG) having one filter disc (0,034m² filter area, 152mm diameter and a pore size of 5 nm), one container as gel reservoir and a second container as buffer reservoir. Both containers were connected to the inlet port of the DCF 152/S via tubing and ball valve to select the respective medium in the different process steps. The double jacket of the housing of the DCF 152/S was connected to a refrigerated circulator to maintain the temperature in the double jacket at a preset temperature of 20°C. The filtrate of the DCF 152/S was collected in a container, placed on a balance to determine the amount of filtrate. The following step was applied:
i) conducting a diafiltration with a buffer solution containing Na₂HPO₄*2H₂O: 5,96 g/L; NaH₂PO₄*2H₂O: 2,57 g/L; NaCl: 2,29 g/L and water for injection by applying pressure (1,5 bar) to the closed buffer container, containing the buffer, open the way to the inlet port of the DCF 152/S and removing the filtrate via the filter discs and hollow shaft (see WO 00/47312). The buffer solution may contain lidocaine (0,27 - 0,33 *w*/*w%).* As an alternative, lidocaine may be added after diafiltration. The filter disc was rotating with 150 rpm (1/min). The gel was filtered until unbound BDDE was present below the limit of quantification (LOQ). The amount of BDDE was determined as explained above (see Example 1). The dynamic filtration process was performed within 2 hours and 1 minute.
The results of further experiments are shown in the following table 2:

**Table 2:**

| Trial | Disc [nm] | Pressure [bar] | T [°C] | Duration [h:m] | BDDE Gel BDDE content at beginning [ppm] | BDDE DCF Final BDDE content [ppm] |
|---|---|---|---|---|---|---|
| 1 | 5 | 1.5 | 20 | 03:17 | 226,8 | below limit of quantification (LOQ) |
| 2 | 30 | 1.5 | 20 | 02:18 | 226.8 | below LOQ |
| 3 | 2000 | 1.5 | 20 | 02:01 | 226.8 | below LOQ |

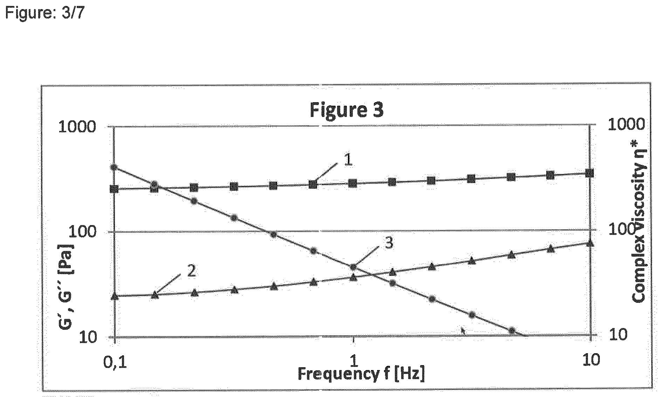

A further example of the fact that the structural stability of the gel was maintained during the method according to the invention can be seen in Figure 3. After the method according to Example 2 was applied, the gel was analyzed with the help of a frequency test which is commonly used for the characterization of gels. The used frequency range was 0.1 to 10 Hz. The parallel increase of G' and G" (G' > G") clearly indicates that the gel ("DCF gel") has maintained its cross-linked structure during the method according to Example 2.

### Example 3: Method of diafiltrating the gel without step of diluting the gel and using dynamic filtration device with six filter discs

The example 3 was carried out following the example 2 described above with the difference that the dynamic filtration device used was a Krauss Maffei Dynamic Crossflow Filter DCF 152/S (Andritz AG) having six (instead of one as in examples 1 and 2) filter disc (0,034m² filter area, 152mm diameter and a pore size of 5 nm). A hyaluronic acid hydrogel, which was cross-linked with BDDE (cf. for example WO 2005/085329) and exhibits a concentration of 66 mg/g, was diluted with the factor 1:2 using a buffer solution as described in examples 1 and 2. The hyaluronic acid hydrogel having a concentration of about 33 mg/g was pumped directly into the process chamber of the dynamic filtration device. The gel contained 226.8 ppm unbound BDDE.

The diafiltration was carried out as described in example 2, wherein the differing process conditions are given in table 3. The diafiltration was carried out until BDDE is present below the limit of quantification and typically until the volume of the filtrate was seven times of the volume of the chamber of the filtration device.

**Table 3**

| Trial | Disc pore size | Number of discs | Pressure | Rotational speed | T | Duration | BDDE Gel | BDDE DCF |
|---|---|---|---|---|---|---|---|---|
| | [nm] | | [bar] | [1/min] | [°C] | [h:m] | [ppm] | [ppm] |
| 4 | 5 | 6 | 2 | 177 | 45 | 04:23 | 226.8 | below LOQ |
| 5 | 5 | 6 | 3 | 77 | 70 | 07:50 | 226.8 | below LOQ |

| | | | | | | | | |
|---|---|---|---|---|---|---|---|---|
| LOQ: limit of quantification BDDE Gel: BDDE content at beginning BDDE DCF: Final BDDE content in the hydrogel after filtration | | | | | | | | |

The structural stability of the gel was maintained during the DCF method according to trials 4 and 5 according to example 3 as shown in the figures 4 (trial 4) and 5 (trial 5). After the method according to Example 3 was applied, the gels were analyzed with the help of a frequency test which is commonly used for the characterization of gels. The used frequency range was 0.1 to 10 Hz. The parallel increase of G' and G" (G' > G") clearly indicates that the gels have maintained their cross-linked structure during the method according to Example 3 (trials 4 and 5).

It was surprisingly found that the structure of the hydrogel is not effected negatively (i.e. the cross-linking structure is maintained), when the number of filter discs as well as the rotational speed were increased compared to example 2. Typically, shear forces increase with increasing number of discs and increasing rotational speed.

## Claims

1. A method for dynamic filtration of a cross-linked biopolymer-based hydrogel comprising the following steps:
a) transferring a cross-linked biopolymer-based hydrogel in a dynamic filtration device which is equipped with semipermeable filter disc(s) and diafiltrating the gel comprising the steps of:
i) concentrating the gel by applying a rotational speed within the range of 20 1/min to 500 1/min and a overpressure within the range of 0.5 to 6 bar to a predetermined concentration; or pumping the gel directly into the process chamber of the dynamic filtration device;
ii) conducting a diafiltration to reduce unwanted molecules, selected from unreactive or unbound polymer cross-linker molecules and their degradation products, by applying a rotational speed within the range of 20 1/min to 500 1/min and a overpressure within the range of 0.5 to 6 bar;
b) optionally adding a mixture comprising a non-cross-linked polymer, in particular non cross-linked hyaluronic acid, and water to the gel,
wherein diafiltration involves adding water or a new buffer to the retentate at the same rate as filtrate is being generated,
wherein the biopolymer-based hydrogel is made of a polymer which is selected from the group consisting of hyaluronic acid, heparosan, alginate, pectin, gellan gum, chondroitin sulfate, keratan, keratan sulfate, heparin, heparin sulfate, cellulose, chitosan, carrageenan, xanthan, or salts or derivatives thereof or combinations thereof, and
wherein the cross-linker bears at least two groups which are capable of reacting with one or more of the hydroxyl groups of the biopolymer-based hydrogel and to cross-link same.

2. The method according to claim 1, wherein the groups of the cross-linker for cross-linking are selected from carboxyl, epoxide, halogen, vinyl, isocyanate or protected isocyanate groups.

3. The method according to claim 1 or 2, wherein the hydrogel is cross-linked by means of a chemical cross-linking agent, and the hydrogel contains a surplus of the chemical cross-linking agent, wherein surplus of the chemical cross-linking agent and / or other unwanted molecules are removed.

4. The method according to any one of claims 1 to 3, wherein the semipermeable filter disc(s) exhibit a pore size of 5 nm to 2µm, more particularly a pore size of 30 nm to 600 nm, more particularly a pore size of 80 nm to 300 nm, particularly a pore size of 5 nm to 60 nm.

5. The method according to any one of claims 1 to 4, wherein the dynamic filtration device is equipped with 1 to 10 semipermeable filter disc(s).

6. The method according to any one of claims 1 to 5, wherein the semipermeable filter disc(s) are made of ceramic, metal or polymer material.

7. The method according to any one of claims 1 to 6, wherein the hydrogel is cross-linked by means of BDDE, and the hydrogel contains a surplus of BDDE, wherein surplus of BDDE and / or other unwanted molecules are removed.

8. The method according to any one of claims 1 to 7, wherein the biopolymer-based hydrogel is made of hyaluronic acid, the diafiltration in step ii) is performed by applying a rotational speed within the range of 20 1/min to 500 1/min and a pressure within the range of 0.5 to 3 bar, and the diafiltration in step ii) is conducted at a concentration of the hydrogel in the range of 10 to 70 mg/g.

9. The method according to any one of claims 1 to 8, wherein during or after diafiltration an anesthetic agent is added to the gel.

10. The method according to claim 9, wherein during or after diafiltration lidocaine is added to the gel.

11. The method according to any one of claims 1 to 10, wherein the diafiltration in step ii) and optionally the concentration step i) are performed at a temperature in the range of from 60 °C to 70 °C and for a time period of from 2 to 4 hours.

12. The method according to any one of claims 1 to 11, wherein the gel is sterilized in the device at 121°C to 135°C with a holding time of about 2 to 30 minutes.

13. The method according to any one of claims 1 to 12, wherein the method is conducted in less than 10 hours, in particular in less than 5 hours.

14. A cross-linked biopolymer-based hydrogel obtainable by the method according to one of claims 1 to 13, wherein the amount of unreactive or unbound cross-linker molecules and their degradation products in the hydrogel is below the limit of quantification, measured by gas chromatography, wherein the cross-linker is BDDE.

15. The use of the hydrogel of claim 14 for aesthetic applications, in particular soft tissue augmentation.

## Patentansprüche

1. Verfahren zur dynamischen Filtration eines vernetzten Hydrogels auf Biopolymerbasis, umfassend die folgenden Schritte:
a) Überführen eines vernetzten Hydrogels auf Biopolymerbasis in eine dynamische Filtrationsvorrichtung, die mit einer oder mehreren semipermeablen Filterscheiben ausgestattet ist, und Diafiltrieren des Gels, umfassend die folgenden Schritte:
i) Konzentrieren des Gels durch Anlegen einer Rotationsgeschwindigkeit im Bereich von 20 1/min bis 500 1/min und eines Überdrucks im Bereich von 0,5 bis 6 bar auf eine vorbestimmte Konzentration; oder direktes Pumpen des Gels in die Prozesskammer der dynamischen Filtrationsvorrichtung;
ii) Durchführen einer Diafiltration, um unerwünschte Moleküle, ausgewählt aus nicht reaktiven oder ungebundenen Polymervernetzer-Molekülen und deren Abbauprodukten, zu senken, durch Anlegen einer Rotationsgeschwindigkeit im Bereich von 20 1/min bis 500 1/min und eines Überdrucks im Bereich von 0,5 bis 6 bar;
b) optional Zugeben einer Mischung umfassend ein nicht vernetztes Polymer, insbesondere nicht vernetzte Hyaluronsäure, und Wasser zu dem Gel,
wobei die Diafiltration das Hinzufügen von Wasser oder neuem Puffer zum Retentat mit derselben Geschwindigkeit, wie Filtrat erzeugt wird, umfasst,
wobei das Hydrogel auf Biopolymerbasis aus einem Polymer hergestellt ist, ausgewählt aus der Gruppe bestehend aus Hyaluronsäure, Heparosan, Alginat, Pektin, Gellan Gum, Chondroitinsulfat, Keratan, Keratansulfat, Heparin, Heparinsulfat, Cellulose, Chitosan, Carrageen, Xanthan oder Salzen oder Derivaten davon oder Kombinationen davon, und
wobei der Vernetzer mindestens zwei Gruppen trägt, die mit einer oder mehreren der Hydroxylgruppen des Hydrogels auf Biopolymerbasis reagieren und diese vernetzen können.

2. Verfahren nach Anspruch 1, wobei die Gruppen des Vernetzers zur Vernetzung aus Carboxyl-, Epoxid-, Halogen-, Vinyl-, Isocyanat- oder geschützten Isocyanatgruppen ausgewählt sind.

3. Verfahren nach Anspruch 1 oder 2, wobei das Hydrogel mittels eines chemischen Vernetzungsmittels vernetzt ist und das Hydrogel einen Überschuss an chemischem Vernetzungsmittel enthält, wobei der Überschuss an chemischem Vernetzungsmittel und/oder andere unerwünschte Moleküle entfernt werden.

4. Verfahren nach einem der Ansprüche 1 bis 3, wobei die semipermeablen Filterscheiben eine Porengröße von 5 nm bis 2 µm, insbesondere eine Porengröße von 30 nm bis 600 nm, insbesondere eine Porengröße von 80 nm bis 300 nm, insbesondere eine Porengröße von 5 nm bis 60 nm aufweisen.

5. Verfahren nach einem der Ansprüche 1 bis 4, wobei die dynamische Filtrationsvorrichtung mit 1 bis 10 semipermeablen Filterscheiben ausgestattet ist.

6. Verfahren nach einem der Ansprüche 1 bis 5, wobei die semipermeablen Filterscheiben aus Keramik, Metall oder Polymermaterial hergestellt sind.

7. Verfahren nach einem der Ansprüche 1 bis 6, wobei das Hydrogel mittels BDDE vernetzt ist und das Hydrogel einen Überschuss an BDDE enthält, wobei der Überschuss an BDDE und/oder anderen unerwünschten Molekülen entfernt wird.

8. Verfahren nach einem der Ansprüche 1 bis 7, wobei das Hydrogel auf Biopolymerbasis aus Hyaluronsäure hergestellt ist, die Diafiltration in Schritt ii) unter Anwendung einer Rotationsgeschwindigkeit im Bereich von 20 1/min bis 500 1/min und eines Drucks im Bereich von 0,5 bis 3 bar durchgeführt wird und die Diafiltration in Schritt ii) bei einer Konzentration des Hydrogels im Bereich von 10 bis 70 mg/g durchgeführt wird.

9. Verfahren nach einem der Ansprüche 1 bis 8, wobei während oder nach der Diafiltration ein Anästhetikum zu dem Gel hinzugefügt wird.

10. Verfahren nach Anspruch 9, wobei während oder nach der Diafiltration Lidocain zu dem Gel hinzugefügt wird.

11. Verfahren nach einem der Ansprüche 1 bis 10, wobei die Diafiltration in Schritt ii) und optional der Konzentrationsschritt i) bei einer Temperatur im Bereich von 60 °C bis 70 °C und über einen Zeitraum von 2 bis 4 Stunden durchgeführt werden.

12. Verfahren nach einem der Ansprüche 1 bis 11, wobei das Gel in der Vorrichtung bei 121 °C bis 135 °C mit einer Haltezeit von etwa 2 bis 30 Minuten sterilisiert wird.

13. Verfahren nach einem der Ansprüche 1 bis 12, wobei das Verfahren in weniger als 10 Stunden, insbesondere in weniger als 5 Stunden, durchgeführt wird.

14. Ein vernetztes Hydrogel auf Biopolymerbasis, erhältlich durch das Verfahren gemäß einem der Ansprüche 1 bis 13, wobei die Menge an unreaktiven oder ungebundenen Vernetzer-Molekülen und deren Abbauprodukten im Hydrogel unterhalb der Quantifizierungsgrenze liegt, gemessen durch Gaschromatographie, wobei der Vernetzer BDDE ist.

15. Verwendung des Hydrogels nach Anspruch 14 für ästhetische Anwendungen, insbesondere zur Weichgewebeaugmentation.

## Revendications

1. Procédé de filtration dynamique d'un hydrogel à base de biopolymère réticulé comprenant les étapes suivantes :
a) transfert d'un hydrogel à base de biopolymère réticulé dans un dispositif de filtration dynamique qui est équipé de disque(s) filtrant(s) semi-perméable(s) et diafiltration du gel comprenant les étapes de :
i) concentration du gel par application d'une vitesse de rotation dans la plage de 20 1/min à 500 1/min et une surpression dans la plage de 0,5 à 6 bars jusqu'à une concentration prédéterminée ; ou pompage du gel directement dans la chambre de processus du dispositif de filtration dynamique ;
ii) conduite d'une diafiltration pour réduire les molécules indésirables, choisies parmi des molécules d'agent de réticulation de polymère non réactives ou non liées et leurs produits de dégradation, par application d'une vitesse de rotation dans la plage de 20 1/min à 500 1/min et d'une surpression dans la plage de 0,5 à 6 bars ;
b) éventuellement ajout d'un mélange comprenant un polymère non réticulé, en particulier un acide hyaluronique non réticulé, et de l'eau au gel, dans lequel la diafiltration met en oeuvre l'ajout d'eau ou d'un nouveau tampon au rétentat à la même vitesse que le filtrat est généré,
dans lequel l'hydrogel à base de biopolymère est composé d'un polymère qui est choisi dans le groupe constitué d'acide hyaluronique, d'héparosane, d'alginate, de pectine, de gomme gellane, de sulfate de chondroïtine, de kératane, de sulfate de kératane, d'héparine, de sulfate d'héparine, de cellulose, de chitosane, de carraghénane, de xanthane ou leurs sels ou dérivés ou des combinaisons de ceux-ci, et
dans lequel l'agent de réticulation porte au moins deux groupes qui sont capables de réagir avec un ou plusieurs des groupes hydroxyle de l'hydrogel à base de biopolymère et de réticuler celui-ci.

2. Procédé selon la revendication 1, dans lequel les groupes de l'agent de réticulation pour la réticulation sont choisis parmi les groupes carboxyle, époxyde, halogène, vinyle, isocyanate ou isocyanate protégé.

3. Procédé selon la revendication 1 ou 2, dans lequel l'hydrogel est réticulé au moyen d'un agent de réticulation chimique, et l'hydrogel contient un surplus de l'agent de réticulation chimique, dans lequel le surplus de l'agent de réticulation chimique et/ou d'autres molécules indésirables sont éliminé(e)s.

4. Procédé selon l'une quelconque des revendications 1 à 3, dans lequel le ou les disques filtrants semi-perméables présentent une taille de pore de 5 nm à 2 µm, plus particulièrement une taille de pore de 30 nm à 600 nm, plus particulièrement une taille de pore de 80 nm à 300 nm, plus particulièrement une taille de pore de 5 nm à 60 nm.

5. Procédé selon l'une quelconque des revendications 1 à 4, dans lequel le dispositif de filtration dynamique est équipé de 1 à 10 disques filtrants semi-perméables.

6. Procédé selon l'une quelconque des revendications 1 à 5, dans lequel le ou les disques filtrants semi-perméables sont faits de céramique, de métal ou de matériau de polymère.

7. Procédé selon l'une quelconque des revendications 1 à 6, dans lequel l'hydrogel est réticulé au moyen de BDDE, et l'hydrogel contient un surplus de BDDE, dans lequel le surplus de BDDE et/ou d'autres molécules indésirables sont éliminé(e)s.

8. Procédé selon l'une quelconque des revendications 1 à 7, dans lequel l'hydrogel à base de biopolymère est composé d'acide hyaluronique, la diafiltration dans l'étape ii) est réalisée par application d'une vitesse de rotation dans la plage de 20 1/min à 500 1/min et une pression dans la plage de 0,5 à 3 bars, et la diafiltration dans l'étape ii) est réalisée à une concentration de l'hydrogel dans la plage de 10 à 70 mg/g.

9. Procédé selon l'une quelconque des revendications 1 à 8, dans lequel, pendant ou après la diafiltration, un agent anesthésique est ajouté au gel.

10. Procédé selon la revendication 9, dans lequel, pendant ou après la diafiltration, de la lidocaïne est ajoutée au gel.

11. Procédé selon l'une quelconque des revendications 1 à 10, dans lequel la diafiltration dans l'étape ii) et éventuellement l'étape de concentration i) sont effectuées à une température dans la plage de 60 °C à 70 °C et pendant une période de temps de 2 à 4 heures.

12. Procédé selon l'une quelconque des revendications 1 à 11, dans lequel le gel est stérilisé dans le dispositif à une température de 121 °C à 135 °C avec un temps de maintien d'environ 2 à 30 minutes.

13. Procédé selon l'une quelconque des revendications 1 à 12, dans lequel le procédé est conduit en moins de 10 heures, en particulier en moins de 5 heures.

14. Hydrogel à base de biopolymère réticulé pouvant être obtenu par le procédé selon l'une des revendications 1 à 13, dans lequel la quantité de molécules d'agent de réticulation non réactives ou non liées et leurs produits de dégradation dans l'hydrogel est inférieure à la limite de quantification, mesurée par chromatographie en phase gazeuse, dans lequel l'agent de réticulation est le BDDE.

15. Utilisation de l'hydrogel selon la revendication 14 pour des applications esthétiques, en particulier l'augmentation de tissus mous.
